(19) Europäisches Patentamt
European Patent Office
Office européen des brevets

(11) Veröffentlichungsnummer : **0 144 668**
**B1**

(12) **EUROPÄISCHE PATENTSCHRIFT**

(45) Veröffentlichungstag der Patentschrift : **23.12.87**

(51) Int. Cl.⁴ : **C 07 D253/06**

(21) Anmeldenummer : **84112707.9**

(22) Anmeldetag : **22.10.84**

# (54) Verfahren zur Herstellung von 3,4,6-trisubstituierten 3-Alkylthio-1,2,4-triazin-5-on-Derivaten.

(30) Priorität : **04.11.83 DE 3339859**

(43) Veröffentlichungstag der Anmeldung : **19.06.85 Patentblatt 85/25**

(45) Bekanntmachung des Hinweises auf die Patenterteilung : **23.12.87 Patentblatt 87/52**

(84) Benannte Vertragsstaaten : **AT BE CH DE FR GB IT LI NL**

(56) Entgegenhaltungen :
**EP-A- 0 049 416**
**EP-A- 0 056 938**
**EP-A- 0 058 885**
**EP-A- 0 074 538**
**EP-A- 0 084 774**

(73) Patentinhaber : **BAYER AG**
**Konzernverwaltung RP Patentabteilung**
**D-5090 Leverkusen 1 Bayerwerk (DE)**

(72) Erfinder : **Schmidt, Thomas, Dr.**
**Ginsterweg 9**
**D-5657 Haan (DE)**

**EP 0 144 668 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein neues, technisch einsetzbares Verfahren zur Herstellung von weitgehend bekannten, herbizid wirksamen 3,4,6-trisubstituierten 3-Alkylthio-1,2,4-triazin-5-on-Derivaten, das auf einer Alkylierung der entsprechenden 3-Mercapto-Derivate in stark saurem Medium beruht.

Es ist bereits bekannt geworden, daß man 3,4,6-trisubstituierte 3-Alkylthio-1,2,4-triazin-5-on-Derivate der Formel (IA)

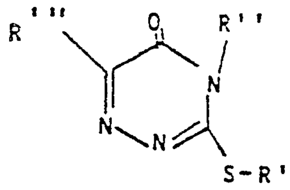

(IA)

in welcher

R′ für Alkyl, Alkenyl, Alkinyl, gegebenenfalls substituiertes Cycloalkyl oder gegebenenfalls substituiertes Aralkyl steht,

R″ für Amino oder Alkyl steht und

R‴ für jeweils gegebenenfalls substituierte aliphatische, cycloaliphatische, aromatische, araliphatische oder heterocyclische Reste steht,

erhält, wenn man entsprechende — in zwei tautomeren Formen auftretende — 3-Mercapto-1,2,4-triazin-5-on-Derivate der Formel (IIA)

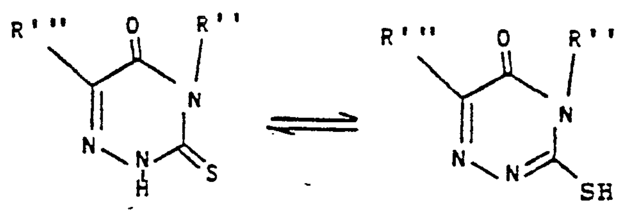

(IIA)

worin R″ und R‴ die oben angegebene Bedeutung haben, mit Alkylierungsmitteln der Formel (IIIA),

Hal—R′ (IIIA)

in welcher

Hal für Halogen steht und

R′ die oben angegebene Bedeutung hat, in alkalischer Lösung bei Temperaturen zwischen 0° und 50 °C umsetzt (vgl. A. Dornow et al., Chem. Ber. 97, S. 2173 bis 8 (1964) ; ferner z. B. DE-A-1 542 873, US-A-3 544 570, US-A-3 671 523).

Der Ausdruck « 3-Alkylthio- » schließt gemäß obiger Definition des Restes R′ auch Alkenyl-, Alkinyl-, Cycloalkyl- und Aralkylthio-Reste sowie zum Teil deren Substitutionsderivate ein ; Entsprechendes gilt für den obigen Ausdruck « Alkylierungsmittel der Formel (IIIA) ».

Dieses vorbekannte Verfahren weist jedoch eine Reihe von Nachteilen auf. So sind große Volumina an Natronlauge zum Lösen der Verbindungen der Formel (IIA) erforderlich, was zu ungünstigen Raum/Zeit-Ausbeuten führt. Weiterhin ist das Auftreten von Halogenid, insbesondere von Bromid oder Iodid, im Zusammenhang mit der Abwasserbelastung, insbesondere beim Arbeiten im technischen Maßstab, nicht unbedenklich. In vielen Fällen führt die Verwendung von Alkylierungsmitteln der Formel (IIIA), wie z. B. Methyliodid oder -bromid, in nicht unbeträchtlichem Maß zu unerwünschter N-Alkylierung am N-2 ; die Bildung dieser unerwünschten, nicht herbizid wirksamen Nebenprodukte ist als Hauptnachteil des bekannten Verfahrens anzusehen. Methyliodid hat außerdem den Nachteil eines sehr teuren Alkylierungsmittels und Methylbromid den Nachteil eines tiefen Siedepunktes, was bei einer technischen Handhabung zu Problemen führt.

Es wurde nun überraschend gefunden, daß man die 3,4,6-trisubstituierten 3-Alkylthio-1,2,4-triazin-5-on-Derivate der Formel (I),

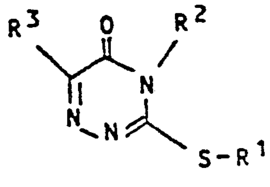

(I)

in welcher

$R^1$ für geradkettiges oder verzweigtes Alkyl mit 1-12 C-Atomen, für jeweils geradkettiges oder verzweigtes Alkenyl oder Alkinyl mit jeweils 2-6 C-Atomen, für Cycloalkyl mit 5-7 C-Atomen (welches einfach bis dreifach, gleich oder verschieden durch Alkyl mit 1-4 C-Atomen oder Halogen substituiert sein kann) oder für Phenylalkyl mit 1-4 C-Atomen im Alkylteil (welches im Phenylteil einfach bis dreifach, gleich oder verschieden durch Halogen, Alkyl mit 1-4 C-Atomen, Alkoxy oder Alkylthio mit jeweils 1-2 C-Atomen, Nitro, Cyano, Halogenalkyl, Halogenalkoxy oder Halogenalkylthio mit jeweils 1-2 C-Atomen und 1-5 gleichen oder verschiedenen Halogenatomen, durch jeweils gegebenenfalls halogen-substituiertes Phenyl oder Phenoxy substituiert sein kann), steht,

$R^2$ für Amino, für Alkylamino mit 1-4 C-Atomen oder für geradkettiges oder verzweigtes Alkyl mit 1-4 C-Atomen steht und

$R^3$ für geradkettiges oder verzweigtes Alkyl mit 1-8 C-Atomen, für Halogenalkyl mit 1-8 C-Atomen und 1-4 gleichen oder verschiedenen Halogenatomen, für Alkoxyalkyl mit 1-4 C-Atomen in jedem Alkylteil, für Cycloalkyl mit 3-7 C-Atomen (welches einfach bis dreifach, gleich oder verschieden durch Alkyl mit 1-4 C-Atomen oder Halogen substituiert sein kann), oder für jeweils im Phenylteil einfach bis dreifach, gleich oder verschieden substituiertes Phenylalkyl (mit 1-4 C-Atomen im Alkylteil) oder Phenyl steht, wobei jeweils als Phenylsubstituenten die bei $R^1$ genannten Phenylsubstituenten möglich sind,

auch in technischem Maßstab in guter Ausbeute und Reinheit erhält, wenn man 3-Mercapto-1,2,4-triazin-5-on-Derivate der Formel (II)

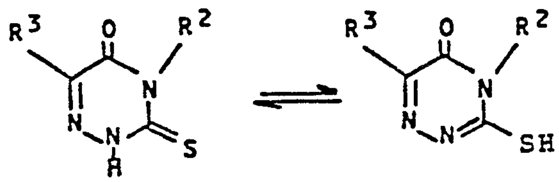

(II)

worin $R^2$ und $R^3$ die oben angegebene Bedeutung haben, bei einer Temperatur zwischen —20 °C und 180 °C mit Estern von Sauerstoffsäuren, ausgewählt aus der Gruppe bestehend aus

a) Mono- oder Diestern der Schwefelsäure der Formeln

$$R^1O—SO_2—OH \quad (IIIa)$$

und

$$R^1O—SO_2—OR^1 \quad (IIIb),$$

b) Mono-, Di- und Triestern der Phosphorsäure der Formeln

$$R^1O—PO(OH)_2 \quad (IVa)$$

und

$$(R^1O)_2PO—OH \quad (IVb)$$

und

$$(R^1O)_3PO \quad (IVc),$$

c) Mono-, Di- und Triestern der phosphorigen Säure der Formeln

$$R^1O—P(OH)_2 \quad (Va)$$

und

$$(R^1O)_2P—OH \quad (Vb)$$

und

$$(R^1O)_3P \quad (Vc),$$

in welchen jeweils $R^1$ die oben angegebene Bedeutung hat,

d) Sulfonsäureestern der Formel

$$R{-}SO_2OR^1 \qquad (VI)$$

in welcher
$R^1$ die oben angegebene Bedeutung hat und
R für Alkyl (insbesondere Methyl), gegebenenfalls substituiertes Phenyl (insbesondere Methylphenyl) oder Halogen (insbesondere Chlor) steht,
e) Chlorkohlensäureestern der Formel

$$Cl{-}CO{-}OR^1 \qquad (VII)$$

in welcher $R^1$ die oben angegebene Bedeutung hat ;
f) Chlorsulfinsäureestern der Formel

$$Cl{-}SO{-}OR^1 \qquad (VIII)$$

in welcher $R^1$ die oben angegebene Bedeutung hat ;
g) Sulfinsäureestern der Formel

$$R^1O{-}SO{-}OR^1 \qquad (IX)$$

in welcher $R^1$ die oben angegebene Bedeutung hat ;
h) Diestern der Dischwefelsäure der Formel

$$R^1O{-}SO_2{-}O{-}SO_2{-}OR^1 \qquad (X)$$

in welcher $R^1$ die oben angegebene Bedeutung hat ;
und
i) Alkylestern von stark sauren Ionenaustauschern, in Gegenwart der mindestens äquimolaren Menge einer starken organischen oder anorganischen sauerstoffhaltigen Säure mit einem $pK_s$-Wert $< 2$ alkyliert, wobei die als Alkylierungsmittel verwendeten Ester auch in situ erzeugt werden können.

Der Ausdruck « 3-Alkylthio- » schließt gemäß obiger Definition des Restes $R^1$ wiederum auch Alkylderivate, nämlich Alkenyl-, Alkinyl-, Cycloalkyl- und Aralkyl-thio-Reste sowie zum Teil deren Substitutionsderivate ein ; Entsprechendes gilt auch für die obigen Ausdrücke « alkyliert », « Alkylierungsmittel » und « Alkylierung ».

Es ist als ausgesprochen überraschend zu bezeichnen, daß sich die 3,4,6-trisubstituierten 3-Alkylthio-1,2,4-triazin-5-on-Derivate der Formel (I) durch dieses Verfahren in guter Ausbeute und Reinheit erhalten lassen, weil sich einerseits nicht voraussehen ließ, wie sich die Ausgangsverbindungen der Formel (II) in stark saurem Medium verhalten würden, d. h. ob möglicherweise Ringspaltung oder — besonders bei Verwendung von konzentrierter Schwefelsäure — Oxidationsreaktionen eintreten würden, oder ob und gegebenenfalls in welcher Position eine Protonierung stattfinden würde. Da das Ringgerüst unter den Reaktionsbedingungen unverändert erhalten bleibt, dürfte im stark sauren Medium tatsächlich eine Protonierung eintreten ; ob hierbei ein einheitliches Zwischenprodukt gebildet wird oder nicht, ist bisher nicht bekannt. Andererseits konnte nicht erwartet werden, daß die Verbindungen der Formel (II) nach erfolgter Protonierung noch alkyliert werden können, und noch viel weniger, daß dazu Ester von Sauerstoffsäuren hervorragend geeignet sind. Überraschend ist außerdem, daß die Endprodukte unter den stark sauren Reaktionsbedingungen stabil sind, zumal eigene Versuche gezeigt hatten, daß die Verbindungen der Formel (I) in verdünnten (z. B. 10 %igen) wäßrigen Säuren zur Hydrolyse neigen.

Das erfindungsgemäße Verfahren hat den Vorteil einer technisch einfachen Durchführung mit hoher Raum/Zeit-Ausbeute. Die verwendeten Alkylierungsmittel führen zu keinen Abwasserproblemen und sind weitgehend preiswerte Substanzen. Von besonderem Vorteil ist jedoch, daß die Endprodukte der Formel (I) frei sind von den an N-2 alkylierten, unerwünschten isomeren Nebenprodukten, welche bei dem vorbekannten Verfahren zwangsläufig anfallen.

Die nach dem erfindungsgemäßen Verfahren herstellbaren 3,4,6-trisubstituierten 3-Alkylthio-1,2,4-triazin-5-on-Derivate sind durch die Formel (I) allgemein definiert.

Besonders bevorzugt sind diejenigen Verbindungen der Formel (I), in denen
$R^1$ für Methyl oder Ethyl steht,
$R^2$ für Amino, Methylamino, Methyl oder Ethyl steht und
$R^3$ für geradkettiges oder verzweigtes Alkyl mit 1 bis 6 Kohlenstoffatomen, für einfach oder zweifach durch Fluor, Chlor oder Brom substituiertes tert.-Butyl, für einfach oder zweifach durch Methoxy oder Ethoxy substituiertes tert.-Butyl, für gegebenenfalls einfach bis dreifach, gleich oder verschieden durch Fluor, Chlor, Methyl oder Ethyl substituiertes Cycloalkyl mit 3 bis 7 Kohlenstoffatomen, sowie für jeweils gegebenenfalls einfach bis zweifach, gleich oder verschieden substituiertes Phenyl oder Phenylalkyl mit 1 bis 4 Kohlenstoffatomen im Alkylteil steht, wobei jeweils als Phenylsubstituenten genannt seien : Fluor,

Chlor, Brom, Methyl, Methoxy, Nitro, Trifluormethyl, Trifluormethoxy, Phenyl und Phenoxy.

Verwendet man beispielsweise 4-Amino-3-mercapto-6-tert.-butyl-1,2,4-triazin-5-on und Dimethylsulfat als Ausgangsstoffe und konzentrierte Schwefelsäure als starke sauerstoffhaltige Säure, so kann der Reaktionsablauf nach dem erfindungsgemäßen Verfahren durch das folgende Formelschema wiedergegeben werden :

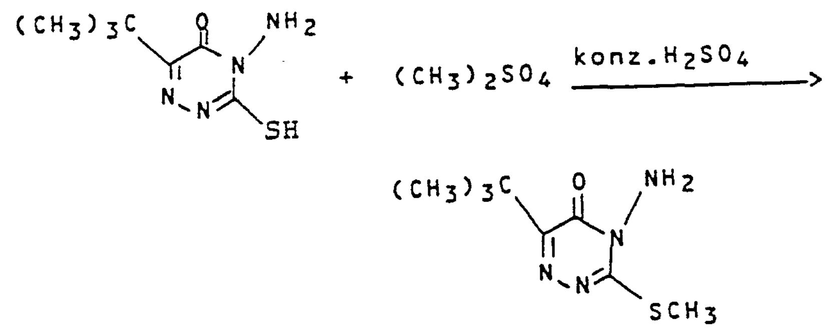


Die bei der Durchführung des erfindungsgemäßen Verfahrens als Ausgangsstoffe zu verwendenden 3-Mercapto-1,2,4-triazin-5-on-Derivate sind durch die obige Formel (II) allgemein definiert. In dieser Formel stehen $R^2$ und $R^3$ vorzugsweise für die Reste, die bereits im Zusammenhang mit der Beschreibung der erfindungsgemäß herstellbaren Stoffe der Formel (I) als besonders bevorzugt für diese Reste genannt wurden.

Die 3-Mercapto-1,2,4-triazin-5-on-Derivate der Formel (II) sind bekannt (vgl. z. B. US-A-3 671 523, US-A-3 544 570, EP-A-0 049 416, EP-A-0 074 538, EP-A-0 084 774, EP-A-0 058 885) bzw. nach den dort angegebenen Verfahren in bekannter Art und Weise erhältlich.

Die Ester der Sauerstoffsäuren der Formeln (III) bis (X) sind allgemein bekannte Verbindungen der organischen Chemie. Sie können entweder als Substanz eingesetzt werden oder in situ aus Sauerstoffsäuren und z. B. Alkoholen, Olefinen, Ethern, Estern, Epoxiden, Carbonaten, Urethanen usw. gebildet werden. Die jeweiligen Bildungsweisen sind bekannte Reaktionen der organischen und anorganischen Chemie. Die unter (i) genannten Alkylester von sauren Ionenaustauschern sind ebenfalls bekannt.

Die erfindungsgemäße Umsetzung wird in Gegenwart einer starken sauerstoffhaltigen Säure (in zumindest äquimolarer Menge) durchgeführt. Hierbei können alle üblicherweise verwendbaren starken anorganischen und organischen Sauerstoffsäuren mit einem $pK_s$-Wert $< 2$ eingesetzt werden, wie insbesondere Schwefelsäure (auch in Form von Oleum), Phosphorsäure, Perchlorsäure, Methansulfonsäure, Methandisulfonsäure, Ethansulfonsäure, Chlorsulfonsäure, Methylschwefelsäure, Ethylschwefelsäure sowie Monochlor-, Dichlor- und Trichloressigsäure. Die Säuren können in reiner, konzentrierter Form oder mit wenig Wasser verdünnt (unter Einhaltung des obigen $pK_s$-Wertes) eingesetzt werden, ferner auch in Form von beliebigen Mischungen miteinander sowie zum Teil auch in Mischung mit starken, nicht sauerstoffhaltigen Mineralsäuren, wie z. B. Chlorwasserstoff ; aus der letztgenannten Gruppe ist als Beispiel die Mischung Eisessig/HCl zu nennen. Darüber hinaus können an Stelle der genannten Säuren auch stark saure Ionenaustauscher — insbesondere solche mit Sulfonsäuregruppen — eingesetzt werden.

Die Reaktionstemperaturen können bei der Durchführung der erfindungsgemäßen Umsetzung in einem größeren Bereich variiert werden. Im allgemeinen arbeitet man — wie oben angegeben — zwischen — 20° und 180 °C, vorzugsweise zwischen 0° und 130 °C, besonders bevorzugt zwischen 10° und 100 °C.

Als Lösungs- bzw. Verdünnungsmittel wird für die erfindungsgemäße Umsetzung mit Vorteil ein Überschuß an jeweils verwendeter starker sauerstoffhaltiger Säure eingesetzt. Die Umsetzung kann jedoch auch in Gegenwart eines inerten organischen Lösungsmittels durchgeführt werden, zum Beispiel dann, wenn als Säure ein saurer Ionenaustauscher verwendet wird. Geeignete Lösungsmittel sind Kohlenwasserstoffe, wie z. B. Benzol, Toluol und Benzinfraktionen ; chlorierte Kohlenwasserstoffe, wie z. B. Dichlormethan, Chloroform, Chlorbenzol und die Dichlorbenzole, ferner Nitroalkane wie z. B. Nitromethan.

Bei der Durchführung der erfindungsgemäßen Umsetzung setzt man auf 1 Mol 3-Mercapto-1,2,4-triazin-5-on-Derivat der Formel (II) 1 bis 10 Äquivalente, vorzugsweise 1 bis 5 Äquivalente an Alkylierungsmittel und 1 bis 30 Mol, vorzugsweise 1 bis 15 Mol, an starker sauerstoffhaltiger Säure ein. Im Falle der Diester der Schwefelsäure, schwefligen Säure, Dischwefelsäure, Phosphorsäure und phosphorigen Säure entspricht dabei 1 Äquivalent an Alkylierungsmittel 0,5 Mol des Diesters, und im Falle der Triester der Phosphorsäure bzw. phosphorigen Säure entspricht 1 Äquivalent an Alkylierungsmittel 0,33 Mol des Triesters.

Hervorzuheben ist, daß das erfindungsgemäße Verfahren kontinuierlich oder diskontinuierlich durchgeführt werden kann. Dabei ist die Reihenfolge der Zugabe der Ausgangsstoffe beliebig.

Die Umsetzung selbst muß nicht bis zu einem vollständigen Umsatz des Ausgangsproduktes

erfolgen. Die Aufarbeitung des Reaktionsgemisches kann mit organischen Lösungsmitteln oder mit Wasser erfolgen.

Nachfolgend wird eine besonders vorteilhafte Ausführungsform des erfindungsgemäßen Verfahrens angegeben : 1 Mol 3-Mercapto-1,2,4-triazin-5-on-Derivat der Formel (II) wird in 2 bis 10 Mol 96 %iger Schwefelsäure aufgeschlämmt und mit 1,1 bis 1,3 Mol eines Alkohols oder eines Chlorsulfonsäurealkylesters versetzt. Man hält die Temperatur 2 bis 5 Stunden bei 10 bis 100 °C, hydrolysiert das Reaktionsgemisch in Wasser und stellt neutral, wobei das Endprodukt der Formel (I) auskristallisiert. Der Feststoff wird abfiltriert, mit Wasser gewaschen und im Vakuum getrocknet.

Es kann jedoch in verschiedenen Fällen auch zweckmäßig sein, zunächst das Alkylierungsmittel mit der Schwefelsäure zu mischen und erst dann das 3-Mercapto-1,2,4-triazin-5-on-Derivat (II) zuzusetzen.

In manchen Fällen — falls ohne organisches Lösungsmittel gearbeitet wurde — erweist es sich als vorteilhaft, das Reaktionsgemisch vor dem Auskristallisieren des Endproduktes mit einem organischen Lösungsmittel, wie z. B. Waschbenzin oder Ligroin, zu versetzen, wodurch auftretende Nebenprodukte in Lösung gehalten werden.

Die erfindungsgemäß herstellbaren Triazinon-Derivate (I) zeichnen sich bekanntermaßen durch sehr gute herbizide Wirksamkeit aus (vgl. z. B. US-A-3 671 523, US-A-3 544 570, EP-A-0 049 416, EP-A-0 074 538, EP-A-0 084 774 und EP-A-0 058 885).

Das erfindungsgemäße Verfahren sei anhand der folgenden Herstellungsbeispiele noch näher erläutert :

## Herstellungsbeispiele

### Beispiel 1a

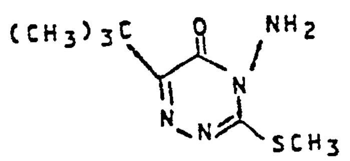

200 g (1 Mol) 4-Amino-3-mercapto-6-tert.-butyl-1,2,4-triazin-5-on werden in 250 g (ca. 2,5 Mol) Schwefelsäure (96 %) gelöst und mit 38,4 g (1,2 Mol) Methanol versetzt. Man erhitzt 4 Stunden auf 80 °C, gibt anschließend auf Eis, neutralisiert mit Natronlauge und stellt einen pH-Wert von 10,5 bis 11,5 ein. Der ausgefallene Niederschlag wird abfiltriert, mit verdünnter Natronlauge und anschließend mit Wasser neutral gewaschen und bei 50 °C im Vakuum getrocknet. Man erhält 171 g 4-Amino-3-methylthio-6-tert.-butyl-1,2,4-triazin-5-on (≙ 94 % der Theorie, bezogen auf verbrauchtes 4-Amino-3-mercapto-6-tert.-butyl-1,2,4-triazin-5-on ; Umsatz 85 %) vom Schmelzpunkt 122 bis 125 °C.

Die vereinigten Mutterlaugen werden mit Säure auf einen pH-Wert von 0,5 bis 1 gebracht ; das ausgefallene, nicht umgesetzte Ausgangsmaterial wird durch Filtration und Trocknung bei 50 °C im Vakuum zurückgewonnen.

Das Endprodukt ist frei von dem Isomeren 4-Amino-2-methyl-3-thioxo-6-tert.-butyl-1,2,4-triazin-5-on.

### Beispiel 1b

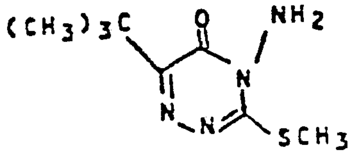

100 g (0,5 Mol) 4-Amino-3-mercapto-6-tert.-butyl-1,2,4-triazin-5-on werden in 250 g (ca. 2,5 Mol) Schwefelsäure (96 %) gelöst und mit 73 g (0,55 Mol) Chlorsulfonsäuremethylester versetzt. Man läßt 4 Stunden bei 20-30 °C reagieren, gibt anschließend aus Eis, neutralisiert mit Natronlauge und stellt einen pH-Wert von 10,5 bis 11,5 ein. Der ausgefallene Niederschlag wird abfiltriert, mit verdünnter Natronlauge und anschließend mit Wasser neutral gewaschen und bei 50 °C im Vakuum getrocknet. Man erhält 94,3 g 4-Amino-3-methylthio-6-tert.-butyl-1,2,4-triazin-5-on (≙ 95 % der Theorie, bezogen auf verbrauchtes 4-Amino-3-mercapto-6-tert.-butyl-1,2,4-triazin-5-on ; Umsatz 95,1 %) vom Schmelzpunkt 122-126 °C.

Die vereingten Mutterlaugen werden mit Säure auf einen pH-Wert von 0,5 bis 1 gebracht ; das ausgefallene, nicht umgesetzte Ausgangsmaterial wird durch Filtration und Trocknung bei 50 °C im Vakuum zurückgewonnen.

Das Endprodukt ist frei von dem Isomeren 4-Amino-2-methyl-3-thioxo-6-tert.-butyl-1,2,4-triazin-5-on.

In entsprechender Weise und gemäß den angegebenen Verfahrensbedingungen können die folgenden Verbindungen der allgemeinen Formel (I)

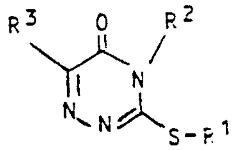 (I)

hergestellt werden :

Tabelle 1

| Bsp. Nr. | $R^1$ | $R^2$ | $R^3$ | Schmelzpunkt (°C) |
|---|---|---|---|---|
| 2 | $CH_3$ | $NH_2$ | $CH_3$ | 165 |
| 3 | $CH_3$ | $NH_2$ | $C_2H_5$ | 119-20 |
| 4 | $CH_3$ | $NH_2$ | $C_3H_7$-i | 123 |
| 5 | $CH_3$ | $NH_2$ | $-CH_2-CH(CH_3)_2$ | 65 |
| 6 | $CH_3$ | $NH_2$ | $-CH_2-C(CH_3)_3$ | 110-11 |
| 7 | $CH_3$ | $NH_2$ | $-CH(CH_3)-CH(CH_3)_2$ | 73-74 |
| 8 | $CH_3$ | $NH_2$ | $-CH(C_2H_5)_2$ | 53-57 |
| 9 | $CH_3$ | $NH_2$ | $-CH(CH_3)-CH_2CH(CH_3)_2$ | 71-75 |
| 10 | $CH_3$ | $NH_2$ | $-CH_2-$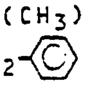 | 166 |
| 11 | $CH_3$ | $NH_2$ | $-CH_2-$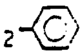$-Cl$ | 183 |
| 12 | $CH_3$ | $NH_2$ | $-CH_2-$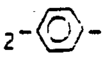$-OCH_3$ | 134-36 |
| 13 | $CH_3$ | $NH_2$ | $-C(CH_3)_2-CH_2F$ | 121-22 |
| 14 | $CH_3$ | $NH_2$ | $-C(CH_3)_2-$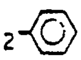 | 119-29 |
| 15 | $CH_3$ | $NH_2$ | $-C(CH_3)_2-CH_2Cl$ | 98 |
| 16 | $CH_3$ | $NH_2$ | $-C(CH_2F)_2CH_3$ | 122-24 |
| 17 | $CH_3$ | $NH_2$ | $-C(CH_3)_2CH_2OCH_3$ | 74-76 |
| 18 | $CH_3$ | $NH_2$ | $-C(CH_3)_2-$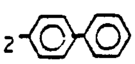 | 180 |
| 19 | $CH_3$ | $NH_2$ | $-C(CH_3)_2-CH_2Br$ | 109-10 |
| 20 | $CH_3$ | $NH_2$ | $-C(CH_3)_2-CH_2OC_2H_5$ | $n_D^{20}$ =1,534 |
| 21 | $CH_3$ | $NH_2$ | 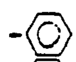$-Cl$ | 182 |
| 22 | $CH_3$ | $NH_2$ | 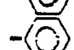$-CH_3$ | 184-85 |
| 23 | $CH_3$ | $NH_2$ | 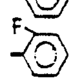 | 147 |
| 24 | $CH_3$ | $NH_2$ | 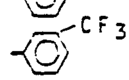 | 156 |
| 25 | $CH_3$ | $NH_2$ | 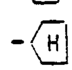 | 140-42 |
| 26 | $CH_3$ | $NH_2$ | 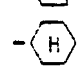 | 152-54 |
| 27 | $CH_3$ | $NH_2$ | 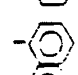 | 188 |
| 28 | $CH_3$ | $NH_2$ | 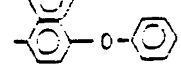 | 185 |

Tabelle 1 (Fortsetzung)

| Bsp. Nr. | $R^1$ | $R^2$ | $R^3$ | Schmelzpunkt (°C) |
|---|---|---|---|---|
| 29 | $CH_3$ | $NH_2$ | 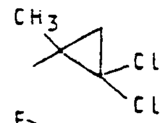 | 133-34 |
| 30 | $CH_3$ | $NH_2$ | 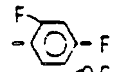 | 198-99 |
| 31 | $CH_3$ | $NH_2$ | 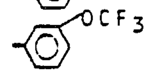 | 153-54 |
| 32 | $CH_3$ | $NH_2$ | 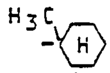 | 117-19 |
| 33 | $CH_3$ | $NH_2$ | 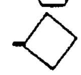 | 118-20 |
| 34 | $C_2H_5$ | $NH_2$ | $-CH_2-C(CH_3)_3$ | 105-11 |
| 35 | $C_2H_5$ | $NH_2$ | $-CH(CH_3)-C_2H_5$ | 74 |
| 36 | $C_2H_5$ | $NH_2$ | $-CH_2-CH(CH_3)_2$ | 79-81 |
| 37 | $C_2H_5$ | $NH_2$ | $-C(CH_3)_2-CH_2F$ | 80-83 |
| 38 | $C_2H_5$ | $NH_2$ | $-C(CH_2F)_2-CH_3$ | 100-03 |
| 39 | $C_2H_5$ | $NH_2$ | $-C(CH_3)_2-CH_2Cl$ | 93-96 |
| 40 | $C_2H_5$ | $NH_2$ | $-(CH_3)_2-CH_2OC_2H_5$ | 80-81 |
| 41 | $C_2H_5$ | $NH_2$ | 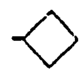 | 140-41 |
| 42 | $C_2H_5$ | $NH_2$ | $-C(CH_3)_2-CH_2OCH_3$ | 83-84 |
| 43 | $C_2H_5$ | $NH_2$ | $-CH(CH_3)_2$ | 113-15 |
| 44 | $C_2H_5$ | $NH_2$ | $-C(CH_3)_3$ | 92-94 |
| 45 | $CH_3$ | $CH_3$ | $CH_3$ | 70 |
| 46 | $CH_3$ | $CH_3$ | $-C(CH_3)_3$ | 96 |
| 47 | $CH_3$ | $CH_3$ | 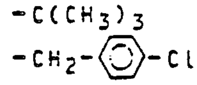 | 140-42 |
| 48 | $CH_3$ | $CH_3$ | $-C(CH_3)_2CH_2F$ | 102-04 |
| 49 | $CH_3$ | $CH_3$ | $-CH_2-C(CH_3)_3$ | 56-59 |
| 50 | $CH_3$ | $CH_3$ | $-C(CH_3)_2-CH(CH_3)_2$ | 103-04 |
| 51 | $CH_3$ | $CH_3$ | $-C(CH_2F)_2-CH_3$ | 104-07 |
| 52 | $CH_3$ | $CH_3$ | 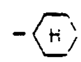 | 115 |
| 53 | $CH_3$ | $CH_3$ | 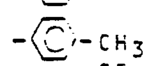 | 136-38 |
| 54 | $CH_3$ | $CH_3$ | 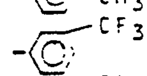 | 125 |
| 55 | $CH_3$ | $CH_3$ | 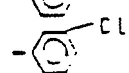 | 139 |
| 56 | $CH_3$ | $CH_3$ | 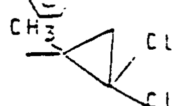 | 110-12 |
| 57 | $CH_3$ | $CH_3$ | 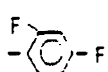 | 117-19 |
| 58 | $CH_3$ | $CH_3$ | 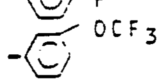 | 83-85 |
| 59 | $CH_3$ | $CH_3$ | 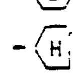 | 72-73 |

Tabelle 1 (Fortsetzung)

| Bsp. Nr. | $R^1$ | $R^2$ | $R^3$ | Schmelzpunkt |
|---|---|---|---|---|
| 60 | $C_2H_5$ | $CH_3$ | 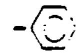 | 69-71 |
| 61 | $C_2H_5$ | $CH_3$ | 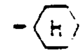 | 94-97 |
| 62 | $C_2H_5$ | $CH_3$ | $-CH_2-CH(CH_3)_2$ | 37-38 |
| 63 | $C_2H_5$ | $CH_3$ | $-CH(CH_3)_2$ | 70-74 |
| 64 | $C_2H_5$ | $CH_3$ | 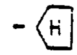 | 50-53 |
| 65 | $C_2H_5$ | $CH_3$ | $-CH_2C(CH_3)_3$ | 46-47 |
| 66 | $C_2H_5$ | $CH_3$ | $-CH(CH_3)C_2H_5$ | 48-49 |
| 67 | $C_2H_5$ | $CH_3$ | $-C(CH_3)_2-CH_2F$ | 74-76 |
| 68 | $C_2H_5$ | $CH_3$ | $-C(CH_2F)_2CH_3$ | 102-04 |
| 69 | $CH_3$ | $C_2H_5$ | $-CH_2-C(CH_3)_3$ | 95-96 |
| 70 | $CH_3$ | $C_2H_5$ | $-CH(CH_3)_2$ | 79-81 |
| 71 | $CH_3$ | $C_2H_5$ | $-C(CH_3)_2-CH_2F$ | 98-99 |
| 72 | $CH_3$ | $C_2H_5$ | $-C(CH_2F)-CH_3$ | 102-05 |
| 73 | $CH_3$ | $C_2H_5$ | 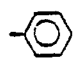 | 78 |
| 74 | $CH_3$ | $C_2H_5$ | 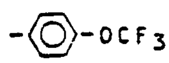 | 122-24 |
| 75 | $C_2H_5$ | $C_2H_5$ | $-C(CH_3)_3$ | 47 |
| 76 | $CH_3$ | $NH_2$ | 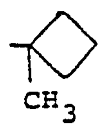 | |
| 77 | $CH_3$ | $NH_2$ | $-C(CH_3)_2-C_2H_5$ | |
| 78 | $CH_3$ | $NH_2$ | 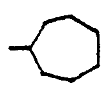 | |
| 79 | $CH_3$ | $NH_2$ | 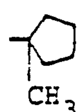 | |
| 80 | $CH_3$ | $NH_2$ | 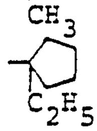 | |

Beispiel 44a

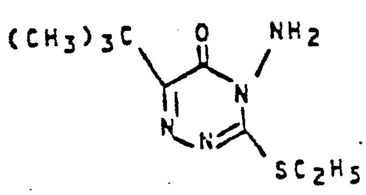

300 g (ca. 3 Mol) Schwefelsäure (100 %ig) werden mit 46 g (1 Mol) Ethanol versetzt. Man kühlt auf 20 °C ab und fügt 200 g (1 Mol) 4-Amino-3-mercapto-6-tert.-butyl-1,2,4-triazin-5-on zu. Anschließend erwärmt man 5 Stunden auf 80 °C, gießt danach das Reaktionsgemisch auf Eis, neutralisiert mit Natronlauge und stellt einen pH-Wert von 10,5-11,5 ein. Der ausgefallene Niederschlag wird abfiltriert, mit verdünnter Natronlauge und anschließend mit Wasser neutral gewaschen und bei 50 °C im Vakuum getrocknet. Man erhält 171,5 g 4-Amino-3-ethylthio-6-tert.-butyl-1,2,4-triazin-5-on (≙ 94,3 % der Theorie, bezogen auf verbrauchtes 4-Amino-3-mercapto-6-tert.-butyl-1,2,4-triazin-5-on ; Umsatz : 79,75 %) vom Schmelzpunkt 93-95 °C.

Die vereinigten Mutterlaugen werden mit Säure auf einen pH-Wert von 0,5 bis 1 gebracht ; das ausgefallene, nicht umgesetzte Ausgangsmaterial wird durch Filtration und Trocknung bei 50 °C im Vakuum zurückgewonnen.

Das Endprodukt ist frei von dem Isomeren 4-Amino-2-ethyl-3-thioxo-6-tert.-butyl-1,2,4-triazin-5-on.

## Patentansprüche

1. Verfahren zur Herstellung von 3,4,6-trisubstituierten 3-Alkylthio-1,2,4-triazin-5-onen der Formel (1)

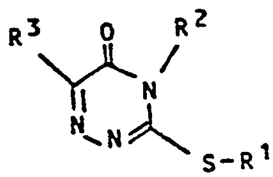

(I)

in welcher

$R^1$ für geradkettiges oder verzweigtes Alkyl mit 1-12 C-Atomen, für jeweils geradkettiges oder verzweigtes Alkenyl oder Alkinyl mit jeweils 2-6 C-Atomen, für Cycloalkyl mit 5-7 C-Atomen (welches einfach bis dreifach, gleich oder verschieden durch Alkyl mit 1-4 C-Atomen oder Halogen substituiert sein kann) oder für Phenylalkyl mit 1-4 C-Atomen im Alkylteil (welches im Phenylteil einfach bis dreifach, gleich oder verschieden durch Halogen, Alkyl mit 1-4 C-Atomen, Alkoxy oder Alkylthio mit jeweils 1-2 C-Atomen, Nitro, Cyano, Halogenalkyl, Halogenalkoxy oder Halogenalkylthio mit jeweils 1-2 C-Atomen und 1-5 gleichen oder verschiedenen Halogenatomen, durch jeweils gegebenenfalls halogen-substituiertes Phenyl oder Phenoxy substituiert sein kann), steht,

$R^2$ für Amino, für Alkylamino mit 1-4 C-Atomen oder für geradkettiges oder verzweigtes Alkyl mit 1-4 C-Atomen steht und

$R^3$ für geradkettiges oder verzweigtes Alkyl mit 1-8 C-Atomen, für Halogenalkyl mit 1-8 C-Atomen und 1-4 gleichen oder verschiedenen Halogenatomen, für Alkoxyalkyl mit 1-4 C-Atomen in jedem Alkylteil, für Cycloalkyl mit 3-7 C-Atomen (welches einfach bis dreifach, gleich oder verschieden durch Alkyl mit 1-4 C-Atomen oder Halogen substituiert sein kann), oder für jeweils im Phenylteil einfach bis dreifach, gleich oder verschieden substituiertes Phenylalkyl (mit 1-4 C-Atomen im Alkylteil) oder Phenyl steht, wobei jeweils als Phenylsubstituenten die bei $R^1$ genannten Phenylsubstituenten möglich sind,

dadurch gekennzeichnet, daß man 3-Mercapto-1,2,4-triazin-5-on-Derivate der Formel (II)

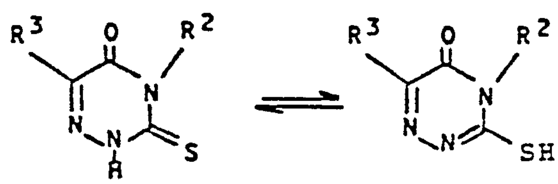

(II)

worin $R^2$ und $R^3$ die oben angegebene Bedeutung haben, bei einer Temperatur zwischen — 20 °C und 180 °C mit Estern von Sauerstoffsäuren, ausgewählt aus der Gruppe bestehend aus

(a) Mono- oder Diestern der Schwefelsäure der Formeln

$$R^1O—SO_2—OH \qquad (IIIa)$$

und

$$R^1O—SO_2—OR^1 \qquad (IIIb),$$

(b) Mono-, Di- und Triestern der Phosphorsäure der Formeln

$$R^1O{-}PO(OH)_2 \qquad (IVa)$$

und

$$(R^1O)_2PO{-}OH \qquad (IVb)$$

und

$$(R^1O)_3PO \qquad (IVc),$$

(c) Mono-, Di- und Triestern der phosphorigen Säure der Formeln

$$R^1O{-}P(OH)_2 \qquad (Va)$$

und

$$(R^1O)_2P{-}OH \qquad (Vb)$$

und

$$(R^1O)_3P \qquad (Vc)$$

in welchen jeweils $R^1$ die oben angegebene Bedeutung hat,

(d) Sulfonsäureestern der Formel

$$R{-}SO_2OR^1 \qquad (VI)$$

in welcher

$R^1$ die oben angegebene Bedeutung hat und

R für Alkyl (insbesondere Methyl), gegebenenfalls substituiertes Phenyl (insbesondere Methylphenyl) oder Halogen (insbesondere Chlor) steht,

(e) Chlorkohlensäureestern der Formel

$$Cl{-}CO{-}OR^1 \qquad (VII)$$

in welcher $R^1$ die oben angegebene Bedeutung hat;

(f) Chlorsulfinsäureestern der Formel

$$Cl{-}SO{-}OR^1 \qquad (VIII)$$

in welcher $R^1$ die oben angegebene Bedeutung hat;

(g) Sulfinsäureestern der Formel

$$R^1O{-}SO{-}OR^1 \qquad (IX)$$

in welcher $R^1$ die oben angegebene Bedeutung hat;

(h) Diestern der Dischwefelsäure der Formel

$$R^1O{-}SO_2{-}O{-}SO_2{-}OR^1 \qquad (X)$$

in welcher $R^1$ die oben angegebene Bedeutung hat;

und

(i) Alkylestern von stark sauren Ionenaustauschern, in Gegenwart der mindestens äquimolaren Menge einer starken organischen oder anorganischen sauerstoffhaltigen Säure mit einem $pK_s$-Wert $< 2$ alkyliert, wobei die als Alkylierungsmittel verwendeten Ester auch in situ erzeugt werden können.

2. Verfahren nach Anspruch 1, dadurch gekennzeichnet, daß man bei einer Temperatur zwischen 0 °C und 130 °C alkyliert.

3. Verfahren nach Anspruch 2, dadurch gekennzeichnet, daß man bei einer Temperatur zwischen 10 °C und 100 °C alkyliert.

4. Verfahren nach Anspruch 1, dadurch gekennzeichnet, daß man pro Mol Ausgangsverbindung der Formel (II) 1 bis 10 Äquivalente des Alkylierungsmittels einsetzt.

5. Verfahren nach Anspruch 1, dadurch gekennzeichnet, daß man pro Mol Ausgangsverbindung der Formel (II) 1 bis 30 Mol der starken sauerstoffhaltigen Säure einsetzt.

6. Verfahren nach Anspruch 1, dadurch gekennzeichnet, daß man als Säuren Schwefelsäure, Phosphorsäure, Perchlorsäure, Methansulfonsäure, Methandisulfonsäure, Ethansulfonsäure, Chlorsulfonsäure, Methylschwefelsäure, Ethylschwefelsäure, Monochlor-, Dichlor- oder Trichloressigsäure oder stark saure Ionenaustauscher einsetzt.

7. Verfahren nach Anspruch 1, dadurch gekennzeichnet, daß man die starken sauerstoffhaltigen Säuren in Form von beliebigen Mischungen miteinander oder in Form von Mischungen mit starken, nicht sauerstoffhaltigen Mineralsäuren, insbesondere mit Chlorwasserstoff, einsetzt.

8. Verfahren nach Anspruch 1, dadurch gekennzeichnet, daß man die als Alkylierungsmittel benötigten Ester von Sauerstoffsäuren in situ aus den Sauerstoffsäuren und Alkoholen, Olefinen, Ethern, Estern, Epoxiden, Carbonaten oder Urethanen erzeugt.

**Claims**

1. Process for the preparation of 3,4,6-trisubstituted 3-alkylthio-1,2,4-triazin-5-ones of the formula (I)

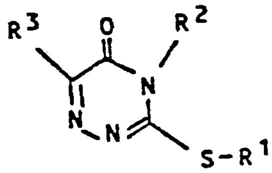

(I)

in which

$R^1$ represents straight-chain or branched alkyl with 1-12 C atoms, alkenyl or alkinyl, each of which is straight-chained or branched and has 2-6 C atoms, cycloalkyl with 5-7 C atoms (which can be mono- to trisubstituted by identical or different substituents from amongst alkyl with 1-4 C atoms or halogen) or phenylalkyl with 1-4 C atoms in the alkyl part (which can be mono- to trisubstituted in the phenyl part by identical or different substituents from amongst halogen, alkyl with 1-4 C atoms, alkoxy or alkylthio with in each case 1-2 C atoms, nitro, cyano, halogenoalkyl, halogenoalkoxy or halogenoalkylthio with in each case 1-2 C atoms and 1-5 identical or different halogen atoms, or by phenyl or phenoxy, each of which is optionally halogen-substituted),

$R^2$ represents amino, alkylamino with 1-4 C atoms or straight-chain or branched alkyl with 1-4 C atoms and

$R^3$ represents straight-chain or branched alkyl with 1-8 C atoms, halogenoalkyl with 1-8 C atoms and 1-4 identical or different halogen atoms, alkoxyalkyl with 1-4 C atoms in each alkyl part, cycloalkyl with 3-7 C atoms (which can be mono- to trisubstituted by identical or different substituents from amongst alkyl with 1-4 C atoms or halogen), or phenylalkyl (with 1-4 C atoms in the alkyl part) or phenyl, each of which is mono- to trisubstituted in the phenyl part by identical or different substituents, possible phenyl substituents in each case being the phenyl substituents mentioned under $R^1$,
characterised in that 3-mercapto-1,2,4-triazin-5-one derivatives of the formula (II)

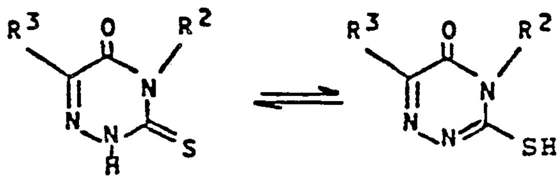

(II)

wherein $R^2$ and $R^3$ have the abovementioned meaning, are alkylated at a temperature between —20 °C and 180 °C with esters of oxo acids, selected from the group consisting of

(a) mono- or diesters of sulphuric acid of the formulae

$$R^1O—SO_2—OH \quad \text{(IIIa)}$$

and

$$R^1O—SO_2—OR^1 \quad \text{(IIIb)},$$

(b) mono-, di- and triesters of phosphoric acid of the formulae

$$R^1O—PO(OH)_2 \quad \text{(IVa)}$$

and

$$(R^1O)_2PO—OH \quad \text{(IVb)}$$

and

$$(R^1O)_3PO \quad \text{(IVc)},$$

(c) mono-, di- and triesters of phosphorous acid of the formulae

$$R^1O—P(OH)_2 \quad (Va)$$

and

$$(R^1O)_2—P—OH \quad (Vb)$$

and

$$(R^1O)_3P \quad (Vc),$$

in each of which $R^1$ has the abovementioned meaning,

(d) sulphonic acid esters of the formula

$$R—SO_2OR^1 \quad (VI)$$

in which

$R^1$ has the abovementioned meaning and

R represents alkyl (in particular methyl), optionally substituted phenyl (in particular methylphenyl) or halogen (in particular chlorine),

(e) chlorocarbonic acid esters of the formula

$$Cl—CO—OR^1 \quad (VII)$$

in which $R^1$ has the abovementioned meaning ;

(f) chlorosulphinic acid esters of the formula

$$Cl—SO—OR^1 \quad (VIII)$$

in which $R^1$ has the abovementioned meaning ;

(g) sulphinic acid esters of the formula

$$R^1O—SO—OR^1 \quad (IX)$$

in which $R^1$ has the abovementioned meaning ;

(h) diesters of disulphuric acid of the formula

$$R^1O—SO_2—O—SO_2—OR^1 \quad (X)$$

in which $R^1$ has the abovementioned meaning ;
and

(i) alkyl esters of strongly acidic ion exchangers, in the presence of an at least equimolar quantity of a strong organic or inorganic oxygen-containing acid with a $pK_a$ value of $< 2$, it also being possible for the esters used as the alkylating agents to be produced in situ.

2. Process according to Claim 1, characterised in that alkylation is carried out at a temperature between 0 °C and 130 °C.

3. Process according to Claim 2, characterised in that alkylation is carried out at a temperature between 10 °C and 100 °C.

4. Process according to Claim 1, characterised in that 1 to 10 equivalents of the alkylating agent are used per mole of the starting compound of the formula (II).

5. Process according to Claim 1, characterised in that 1 to 30 moles of the strong oxygen-containing acid are used per mole of the starting compound of the formula (II).

6. Process according to Claim 1, characterised in that sulphuric acid, phosphoric acid, perchloric acid, methane-sulphonic acid, methanedisulphonic acid, ethanesulphonic acid, chlorosulphonic acid, methylsulphuric acid, ethylsulphuric acid, monochloro-, dichloro- or trichloro-acetic acid or strongly acidic ion exchangers are used as the acids.

7. Process according to Claim 1, characterised in that the strong oxygen-containing acids are used in the form of any desired mixtures with one another or in the form of mixtures with strong mineral acids not containing oxygen, in particular with hydrogen chloride.

8. Process according to Claim 1, characterised in that the esters of oxo acids required as alkylating agents are produced in situ from the oxo acids and alcohols, olefins, ethers, esters, epoxides, carbonates or urethanes.

**Revendications**

1. Procédé de production de 3-alkylthio-1,2,4-triazine-5-ones 3,4,6-trisubstituées de formule (I),

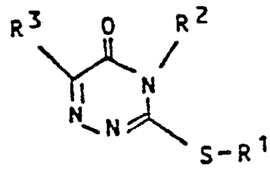

(I)

dans laquelle

$R^1$ est un groupe alkyle à chaîne droite ou ramifiée ayant 1 à 12 atomes de carbone, un groupe alcényle ou alcynyle à chaîne droite ou à chaîne ramifiée ayant chacun 2 à 6 atomes de carbone, un groupe cycloalkyle ayant 5 à 7 atomes de carbone (qui peut porter un à trois substituants alkyle en $C_1$ à $C_4$ ou halogéno identiques ou différents) ou un groupe phénylalkyle ayant 1 à 4 atomes de carbone dans la partie alkyle (qui peut être substitué dans la partie phényle une à trois fois par des substituants, identiques ou différents, halogéno, alkyle ayant 1 à 4 atomes de carbone, alkoxy ou alkylthio ayant chacun un ou deux atomes de carbone, nitro, cyano, halogénalkyle, halogénalkoxy ou halogénalkylthio avec chacun un ou deux atomes de carbone et un à cinq atomes d'halogènes identiques ou différents, un groupe phényle ou phénoxy substitué chacun le cas échéant par un halogène),

$R^2$ est un groupe amino, alkylamino ayant 1 à 4 atomes de carbone, ou un groupe alkyle à chaîne droite ou à chaîne ramifiée ayant 1 à 4 atomes de carbone et

$R^3$ désigne un groupe alkyle à chaîne droite ou ramifiée ayant 1 à 8 atomes de carbone, un groupe halogénalkyle ayant 1 à 8 atomes de carbone et 1 à 4 atomes d'halogènes identiques ou différents, un groupe alkoxyalkyle ayant 1 à 4 atomes de carbone dans chaque partie alkyle, un groupe cycloalkyle ayant 3 à 7 atomes de carbone (qui peut être substitué une à trois fois par des groupes alkyle en $C_1$ à $C_4$ ou des halogènes identiques ou différents) ou un groupe phénylalkyle (avec 1 à 4 atomes de carbone dans la partie alkyle) ou phényle substitués chacun une à trois fois dans la partie phényle par des substituants identiques ou différents, avec dans chaque cas comme substituants possibles du groupe phényle ceux qui sont mentionnés pour $R^1$,

caractérisé par le fait qu'on alkyle en présence de la quantité au moins équimolaire d'un acide organique ou inorganique fort contenant de l'oxygène, avec une valeur de $pK_s$ inférieure à deux, des dérivés de 3-mercapto-1,2,4-triazine-5-one de formule (II)

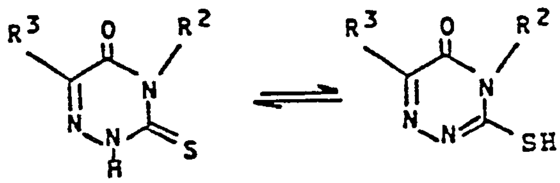

(II)

dans laquelle $R^2$ et $R^3$ ont la définition indiquée ci-dessus, à une température comprise entre —20 et 180 °C, avec des esters d'acides contenant de l'oxygène, choisis dans le groupe comprenant

(a) des monoesters ou diesters de l'acide sulfurique de formules

$$R^1O—SO_2—OH \quad (IIIa)$$

et

$$R^1O—SO_2—OR^1 \quad (IIIb),$$

(b) des monoesters, diesters et triesters d'acide phosphorique de formules

$$R^1O—PO(OH)_2 \quad (IVa)$$

et

$$(R^1O)_2PO—OH \quad (IVb)$$

et

$$(R^1O)_3PO \quad (IVc),$$

c) des monoesters, diesters et triesters d'acide phosphoreux, de formules

$$R^1O—P(OH)_2 \quad (Va)$$

et

$$(R^1O)_2P—OH \quad (Vb)$$

et

$$(R^1O)_3P \quad (Vc)$$

dans chacune desquelles $R^1$ a la définition indiquée ci-dessus,

d) des esters d'acides sulfoniques de formule

$$R—SO_2OR^1 \quad (VI)$$

dans laquelle

$R^1$ a la définition indiquée ci-dessus et

R désigne un groupe alkyle (notamment le groupe méthyle), un groupe phényle éventuellement substitué (notamment le groupe méthylphényle) ou un halogène (notamment le chlore),

e) des esters d'acides chlorocarboniques de formule

$$Cl—CO—OR^1 \quad (VII)$$

dans laquelle $R^1$ a la définition indiquée ci-dessus ;

f) des esters d'acides chlorosulfiniques de formule

$$Cl—SO—OR^1 \quad (VIII)$$

dans laquelle $R^1$ a la définition indiquée ci-dessus ;

g) des esters d'acides sulfiniques de formule

$$R^1O—SO—OR^1 \quad (IX)$$

dans laquelle $R^1$ a la définition indiquée ci-dessus ;

h) des diesters d'acide disulfurique de formule

$$R^1O—SO_2—O—SO_2—OR^1 \quad (X)$$

dans laquelle $R^1$ a la définition indiquée ci-dessus ;

et

i) des esters d'alkyle d'échangeurs d'ions fortement acides, les esters utilisés comme agents alkylants pouvant également être produits in situ.

2. Procédé suivant la revendication 1, caractérisé en ce qu'on alkyle à une température comprise entre 0 et 130 °C.

3. Procédé suivant la revendication 2, caractérisé en ce qu'on alkyle à une température entre 10 et 100 °C.

4. Procédé suivant la revendication 1, caractérisé en ce qu'on utilise par mole de composé de départ de formule (II) 1 à 10 équivalents d'agent alkylant.

5. Procédé suivant la revendication 1, caractérisé en ce qu'on utilise par mole de composé de départ de formule (II) 1 à 30 moles d'acide fort contenant de l'oxygène.

6. Procédé suivant la revendication 1, caractérisé en ce qu'on utilise comme acides l'acide sulfurique, l'acide phosphorique, l'acide perchlorique, l'acide méthanesulfonique, l'acide méthanedisulfonique, l'acide éthanesulfonique, l'acide chlorosulfonique, l'acide méthylsulfurique, l'acide éthylsulfurique, l'acide monochloro, dichloro- ou trichloracétique ou un échangeur ionique fortement acide.

7. Procédé suivant la revendication 1, caractérisé en ce qu'on utilise les acides forts contenant de l'oxygène sous forme de mélanges quelconques les uns avec les autres ou sous la forme de mélanges avec des acides minéraux forts ne contenant pas d'oxygène, en particulier avec le chlorure d'hydrogène.

8. Procédé suivant la revendication 1, caractérisé en ce que les esters d'oxacides nécessaires comme agents d'alkylation sont produits in situ à partir des oxacides et d'alcools, d'oléfines, d'éthers, d'esters, d'époxydes, de carbonates ou d'uréthannes.